(19) Europäisches Patentamt
European Patent Office
Office européen des brevets

(11) Veröffentlichungsnummer: **0 277 352 B1**

# (12) EUROPÄISCHE PATENTSCHRIFT

(45) Veröffentlichungstag der Patentschrift: **16.10.91**

(51) Int. Cl.5: **A61K 31/55**, //(A61K31/55, 31:52,31:135)

(21) Anmeldenummer: **87119095.5**

(22) Anmeldetag: **23.12.87**

Die Akte enthält technische Angaben, die nach dem Eingang der Anmeldung eingereicht wurden und die nicht in dieser Patentschrift enthalten sind.

## (54) Synergistische Kombination von Azelastin und Theophyllin oder Azelastin und Beta-Mimetika.

(30) Priorität: **17.01.87 DE 3701287**

(43) Veröffentlichungstag der Anmeldung: **10.08.88 Patentblatt 88/32**

(45) Bekanntmachung des Hinweises auf die Patenterteilung: **16.10.91 Patentblatt 91/42**

(84) Benannte Vertragsstaaten: **AT BE CH DE ES FR GB GR IT LI LU NL SE**

(56) Entgegenhaltungen:

**ARZNEIMITTEL FORSCHUNG DRUG RESEARCH, Band 31(II), Nr. 8, August 1981, Seiten 1184-1193, Editio Cantor, Verlag für Medizin und Naturwissenschaften KG, Aulendorf, DE; H.-J. ZECHEL et al.: "Pharmacological and toxicological properties of azelastine, a novel antiallergic agent"**

(73) Patentinhaber: **ASTA Pharma Aktiengesellschaft
Weismüllerstrasse 45
W-6000 Frankfurt am Main 1(DE)**

(72) Erfinder: **Achterrath-Tuckermann, Ute, Dr.
Bahnhofstrasse 120A
W-6457 Maintal 1(DE)**
Erfinder: **Aurich, Rudolf, Dr.
Kurt-Schumacher-Strasse 13
W-6367 Karben 4(DE)**
Erfinder: **Engel, Jürgen, Dr.
Erlenweg 3
W-8755 Alzenau(DE)**
Erfinder: **Hettche, Helmut, Dr.
Martinstrasse 23
W-6057 Dietzenbach(DE)**
Erfinder: **Kleemann, Axel, Dr.
Bornweg 26
W-6052 Mühlheim/Main(DE)**

EP 0 277 352 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Beschreibung:

Azelastin ist ein Asthma-protektiver Arzneimittelwirkstoff mit antiallergischen und antihistaminischen Eigenschaften. Sein chemischer Name ist 4-(p-Chlorbenzyl)-2-[N-methyl-perhydroazepinyl-(4)]-1-(2H)-phthalazinon mit der folgenden Strukturformel

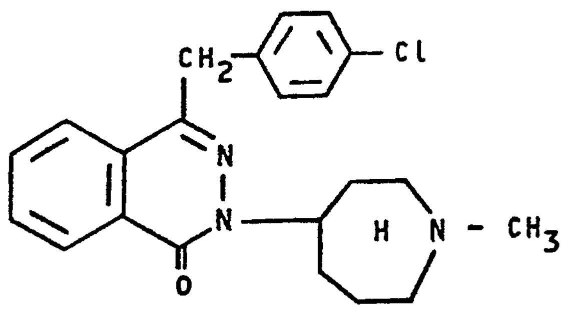

Das Azelastin und dessen Salze mit physiologisch unbedenklichen Säuren besitzen eine ausgeprägte antihistaminische, antiallergische Hauptwirkung sowie eine geringe bronchospasmolytische Wirkungskomponente. Es wurde nun gefunden, daß die Wirkung des Azelastins und seiner Salze überraschenderweise durch Kombination mit Theophyllin oder β-Mimetika und deren Salzen gesteigert wird, wobei gleichzeitig die bronchospasmolytische Wirkung des Theophyllins beziehungsweise die bronchospasmolytische Wirkung des β-Mimetikums ebenfalls eine synergistische Steigerung erfährt. Die Wirkstoffe der erfindungsgemäßen Kombination potenzieren sich also gegenseitig in ihrer Wirkung.

β-Mimetika sind Arzneimittelwirkstoffe mit ausgeprägter bronchospasmolytischer Wirkung. Beispiele hierfür sind: Reproterol (7-{3[(β,3,5-Trihydroxy-phenethyl)-amino]-propyl}-theophyllin), Salbutamol, Terbutalin, Fenoterol, Procaterol, Clenbuterol, Orciprenalin.

Aufgabe der Erfindung ist die Bereitstellung von verbesserten Arzneimitteln mit asthmaprophylaktischer, antiallergischer und antihistaminischer Wirkung für die Humanmedizin sowie für die Veterinärmedizin.

Die Erfindung betrifft Arzneimittel gemäß den Patentansprüchen sowie die Verwendung des Wirkstoffs Azelastin und dessen Salzen mit physiologisch verträglichen Säuren zusammen mit β-Mimetika oder Theophyllin, auch in jeweils getrennten Formulierungen.

Die in den Patentansprüchen angegebenen Gewichtsmengen beziehungsweise Gewichtsteile beziehen sich jeweils auf die reinen Wirkstoffe, das heißt nicht auf Salze dieser Wirkstoffe. Falls Salze verwendet werden, ändern sich die Mengen entsprechend dem geänderten Molgewicht der Salze.

Das Azelastin sowie die β-Mimetika Werden vorzugsweise als Säureadditionssalze verwendet, wobei insbesondere die Salze mit Halogenwasserstoffsäuren (zum Beispiel das Hydrochlorid, Hydrobromid) oder auch mit organischen Säuren (zum Beispiel Embonsäure, Maleinsäure, Citronensäure, Weinsäure) in Frage kommen. Das Theophyllin wird im allgemeinen nicht in Form des Salzes verwendet. Falls es als Salz eingesetzt wird, handelt es sich beispielsweise um ein Salz mit Cholin (2-Hydroxy-N,N,N-trimethylethan). Es besteht jedoch auch die Möglichkeit, Theophyllin in Form eines Molekülassöziates (zum Beispiel mit 2-Amino-2-methylpropanol 1:1 oder Ethanolamin 1:1 oder Magnesiumacetat 1:1 oder N-Methylglucamin 1:1) oder als Gemisch beziehungsweise Verbindung mit Äthylendiamin einzusetzen, wie sie im Europäischen Arzneibuch, Band II, Seite 370-371 oder DAB 9, Seite 1377 - 1378 beschrieben ist.

Die erfindungsgemäße Kombination zeigt beispielsweise im Spasmolyse-Test einen Synergismus der antihistaminischen Wirkung, die gegenüber der antihistaminischen Wirkung des reinen Azelastins und dem reinen Theophyllin oder β-Mimetikum überadditiv gesteigert ist.

Es handelt sich bei der erfindungsgemäßen Kombination um einen überraschenden funktionellen Synergismus. Hierbei tritt sowohl eine synergistisch gesteigerte Antihistamin- bzw. antiallergische als auch eine synergistisch gesteigerte bronchospasmolytische Wirkung auf.

Die Wirkung der erfindungsgemäßen Kombination geht beispielsweise aus folgenden Versuchen hervor. Diese Untersuchungen erfolgten im Histamin-Spasmus-Test am isolierten Meerschweinchen-Ileum. Hierbei wird zum Beispiel die Azelastin-Dosis (Hydrochlorid) konstant gehalten, und die Dosis des Theophyllins beziehungsweise β-Mimetikums (im Beispiel: Reproterol) geändert und jeweils die Hemmung des Darmspasmus in % des ungehemmten Histaminspasmus bestimmt (Methode der Bestimmung des Synergismus nach Magnus).

Beschreibung der Versuchsmethode (isoliertes Meerschweinchenileum):
Meerschweinchen beiderlei Geschlechts im Gewicht von 400-600 g werden durch Genickschlag betäubt und entblutet. Der untere Dünndarm oberhalb der letzten 10 cm vor dem Übergang in den Dickdarm wird herauspräpariert, mit kalter Tyrodelösung durchgespült und 1 Stunde lang im Kühlschrank inkubiert. Anschließend werden 3-4 cm lange Stückchen geschnitten und in die Badgefäße (Tyrodelösung * , 37° C, Carbogenbegasung ** ) unter isotonischen Bedingungen unter 2 g Vorspannung eingehängt. Nach einer Eingewöhnungszeit von ca. 30 Minuten wird die Kontraktion auf eine Standard-Spasmogen-Konzentration Histamin (2 × $10^{-7}$ mol/Liter) ermittelt. Nach Erhalt eines konstanten Standard-Spasmus (+/-10 % vom Mittelwert) wird die spasmolytische Wirkung der vorgesehenen Konzentrationen für beide Kombinationspartner einzeln bestimmt. Anschließend erfolgt am selben Organ die Gabe der Kombination und Bestimmung deren Wirkung. Die Wirkungen der Einzelkomponenten und deren Kombination werden miteinander verglichen.

Die Ergebnisse zeigen die Tabellen 1 und 2

* Tyrodelösung: standardisierte, Glucose-enthaltende physiologische Nährlösung, die den gleichen osmotischen Druck wie Blut besitzt.

** Carbogen: Gasmischung aus 95 % Sauerstoff und 5 % Kohlendioxid.

Tabelle 1

| Azelastin · HCl (µg/ml) | % Hemmung (Histamin 2 x $10^{-10}$ mol/ml) | |
|---|---|---|
| 0,001 * | 38,9 ± 19,9 | N = 8 |
| 0,001 ** | 49,3 ± 1,9 | N = 4 |
| 0,001 *** | 9,4 ± 12,4 | N = 8 |
| 0,001 **** | 46,3 ± 34,6 | N = 8 |
| Theophyllin (µg/ml) | | |
| 10 * | 9,0 ± 12,8 | N = 8 |
| 20 ** | 25,0 ± 15,1 | N = 4 |
| 40 *** | 39,6 ± 26,9 | N = 8 |
| 60 **** | 20,6 ± 15,5 | N = 8 |
| Azelastin · HCl (0,001 µg/ml) + Theophyllin (µg/ml) | | |
| 10 * | 52,6 ± 23,2 | N = 8 |
| 20 ** | 54,8 ± 7,5 | N = 4 |
| 40 *** | 66,4 ± 12,4 | N = 8 |
| 60 **** | 73,1 ± 18,0 | N = 8 |

* kennzeichnen immer die Gruppen, die zusammengehören

N = Anzahl der jeweils verwendeten Tiere

Tabelle 2

| Azelastin • HCl (µg/ml) | | % Hemmung (Histamin 2 x $10^{-10}$ mol/ml) | |
|---|---|---|---|
| 0,001 | * | 35,1 ± 25,7 | N = 8 |
| 0,001 | ** | 26,6 ± 15,1 | N = 8 |
| 0,001 | *** | 45,9 ± 21,1 | N = 8 |
| **Reproterol • HCl (µg/ml)** | | | |
| 0,02 | * | 14,5 ± 16,6 | N = 8 |
| 0,04 | ** | 14,5 ± 18,1 | N = 8 |
| 0,06 | *** | 48,5 ± 21,3 | N = 8 |
| **Azelastin • HCl + Reproterol • HCl (µg/ml)** | | | |
| 0,001 + | | | |
| 0,02 | * | 64,4 ± 16,9 | N = 8 |
| 0,04 | ** | 54,3 ± 13,1 | N = 8 |
| 0,06 | *** | 74,3 ± 24,1 | N = 8 |

* kennzeichnen immer die Gruppen, die zusammengehören
N = Anzahl der jeweils verwendeten Tiere

So wird beispielsweise in dem vorstehend angegebenen Test die antihistaminische Wirkung des Azelastins von 36 % (Mittelwert) um 36 % (Mittelwert) erhöht.

Die antihistaminische Wirkung von Theophyllin wird beispielsweise von 24 % (Mittelwert) durch Zusatz von Azelastin um 38 % (Mittelwert) erhöht.

Die antihistaminische Wirkung von Reproterol wird von 26 % (Mittelwert) durch Zusatz von Azelastin um 38 % (Mittelwert) erhöht.

Die Tagesdosen der erfindungsgemäßen Kombination bestehen zum Beispiel aus 0,1 bis 30 mg,

vorzugsweise 0,5 bis 20 mg und insbesondere 1 bis 10 mg Azelastin und 50 bis 1000 mg, vorzugsweise 80 bis 600 mg, insbesondere i00 bis 500 mg Theophyllin beziehungsweise 0,1 bis 30 mg, vorzugsweise 0,5 bis 20 mg und insbesondere 1 bis 10 mg Azelastin und 0,01 bis 200 mg, vorzugsweise 0,02 bis 100 mg, insbesondere 0,05 bis 50 mg β-Mimetikum.
Die Tagesdosen können in Form einer einmaligen Verabreichung der gesamten Menge oder in Form von 1 bis 10, insbesondere 1 bis 5 Teildosen pro Tag gegeben werden. Im allgemeinen ist eine Verabreichung 1 bis 4 mal, insbesondere 1 bis 3 mal täglich bevorzugt. Beispielsweise beträgt die bevorzugte Dosis für die Kombination von Azelastin und Theophyllin 0,5 bis 10 mg Azelastin und etwa 100 bis 300 mg Theophyllin oder 0,1 bis 10 mg Azelastin und 0,01 bis 20 mg Reproterol, 1 bis 4 mal täglich. Insbesondere beträgt diese Dosis etwa 4 mg Azelastin und etwa 250 mg Theophyllin beziehungsweise 4 mg Azelastin und 10 mg Reproterol 1 bis 3 mal täglich.

Azelastin und Theophyllin werden erfindungsgemäß beispielsweise in folgenden Gewichtsverhältnissen verwendet: 1 Gewichtsteil Azelastin wird mit 3 bis 5000 Gewichtsteilen Theophyllin, vorzugsweise 1 Gewichtsteil Azelastin mit 4 bis 3000 Gewichtsteilen Theophyllin, insbesondere 1 Gewicntsteil Azelastin mit 20 bis 1000 Gewicntsteilen Theophyllin verwendet beziehungsweise kombiniert.

Azelastin und das β-Mimetikum (beziehungsweise eine Mischung verschiedener β-Mimetika) werden erfindungsgemäß in folgenden Gewichtsverhältnissen verwendet: 1 Gewichtsteil Azelastin wird mit 0,0003 bis 1000, vorzugsweise 0,001 bis 250, insbesondere 0,005 bis 40 Gewichtsteilen β-Mimetikum angewendet beziehungsweise kombiniert. Für Reproterol gilt beispielsweise: 1 Gewichtsteil Azelastin wird mit 0,0003 bis 500 Gewichtsteilen, vorzugsweise 0,005 bis 100 Gewichtsteilen, insbesondere mit 0,05 bis 50 Gewichtsteilen Reproterol angewendet beziehungsweise kombiniert.

Beispielsweise lassen sich für die Kombination 10 bis 500 mg Theophyllin und 0,05 bis 30 mg Azelastin, vorzugsweise 100 bis 400 mg Theophyllin und 0,2 bis 15 mg Azelastin, insbesondere 100 bis 300 mg Theophyllin und 0,5 bis 10 mg Azelastin leicht zum Arzneimittel formulieren.

Beispielsweise lassen sich für die Kombination 0,005 bis 200 mg β-Mimetikum (wie zum Beispiel 0,005 - 50 mg Reproterol) und 0,1 bis 30 mg Azelastin, vorzugsweise 0,01 bis 150 mg β-Mimetikum (wie zum Beispiel 0,03 bis 30 mg Reproterol) und 0,3 bis 20 mg Azelastin, insbesondere 0,03 bis 100 mg β-Mimetikum (wie zum Beispiel 0,1 bis 20 mg Reproterol) und 0,5 bis 10 mg Azelastin leicht zum Arzneimittel formulieren.

Diese zuvor angegebenen Gewichtsmengen gelten insbesondere für homogene Mischungen von Theophyllin und Azelastin beziehungsweise β-Mimetika und Azelastin (zum Beispiel Suppositorien oder Einschichttablette). Bei anderen Formülierungen, zum Beispiel Kapseln und Zweischichttabletten, können die Komponenten natürlich auch in anderen Gewichtsmengen zusammen kombiniert werden.

Die Dosierungseinheit der erfindungsgemäßen Kombination kann beispielsweise enthalten:

a) Bei peroralen Arzneiformen:

0,1 bis 30 mg Azelastin, vorzugsweise 0,5 bis 20 mg, insbesondere 1 bis 10 mg Azelastin und 0,005 bis 100 mg, vorzugsweise 0,01 bis 50 mg, insbesondere 0,05 bis 30 mg β-Mimetikum, wie zum Beispiel 1 bis 50 mg, vorzugsweise 5 bis 40 mg, insbesondere 10 bis 20 mg Reproterol.

Für Fenoterol und Terbutalin kommen beispielsweise folgende Mengen in Frage: 0,1 bis 20, vorzugsweise 0,5 bis 10, insbesondere 1 bis 7 mg (Azelastinmenge wie zuvor angegeben).

Für Salbutamol kommen beispielsweise folgende Mengen in Frage: 0,01 bis 10, vorzugsweise 0,05 bis 1, insbesondere 0,1 bis 0,8 mg; bei Retardpräparaten (und auch Zäpfchen) zum Beispiel 0,1 bis 15, vorzugsweise 0,5 bis 10 mg (Azelastinmenge wie zuvor angegeben).

Für Clenbuterol kommen beispielsweise folgende Mengen in Frage: 0,005 bis 1, vorzugsweise 0,01 bis 0,1, insbesondere 0,01 bis 0,05 mg (Azelastinmenge wie zuvor angegeben).

Diese Dosen können beispielsweise 1 bis 5, vorzugsweise 1 bis 4, insbesondere 1 bis 3 mal täglich verabreicht werden.

b) Bei parenteralen Arzneiformen (zum Beispiel intravenös intramuskulär; pro 1 ml):

0,05 bis 30 mg Azelästin, vorzugsweise 0,1 bis 20 mg, insbesondere 0,2 bis 10 mg oder auch 0,3 bis 5 mg Azelastin und 0,01 bis 5 mg, vorzugsweise 0,03 bis 1 mg, insbesondere 0,05 bis 0,5 mg β-Mimetikum, wie zum Beispiel 0,01 bis 1 mg, vorzugsweise 0,03 bis 0,5 mg, insbesondere 0,05 bis 0,1 mg Reproterol.

Für Fenoterol und Terbutalin kommen beispielsweise folgende Mengen in Frage: 0,01 bis 5, vorzugsweise 0,1 bis 1, insbesondere 0,2 bis 0,7 mg (Azelastinmenge wie zuvor angegeben).

Diese Dosen können beispielsweise 1 bis 5, vorzugsweise 1 bis 4, insbesondere 1 bis 3 mal täglich verabreicht werden.

c) Bei Arzneiformen zur Applikation auf die Haut und Schleimhäute (zum Beispiel als Lösungen, Lotionen, Emulsionen, Salben, Pflaster und so weiter):

5 bis 50 mg Azelastin, vorzugsweise 5 bis 30 mg, insbesondere 10 bis 20 mg Azelastin und 10 bis 200 mg, vorzugsweise 25 bis 100 mg, insbesondere 40 bis 80 mg ß-Mimetikum, wie zum Beispiel 10 bis 100 mg, vorzugsweise 25 bis 75 mg, insbesondere 30 bis 60 mg Reproterol.

Diese Dosen können beispielsweise 1 bis 5, vorzugsweise 1 bis 4, insbesondere 1 bis 2 mal täglich verabreicht werden.

d) Bei Arneiformen zum Inhalieren (zum Beispiel in Form von Aerosolen oder Stäubepulvern; pro Aerosolstoß):

0,1 bis 10 mg Azelastin, vorzugsweise 0,1 bis 5 mg, insbesondere 0,1 bis 3 mg Azelastin und 0,005 bis 100 mg, vorzugsweise 0,008 bis 8 mg, insbesondere 0,01 bis 5 mg $\beta$-Mimetikum, wie zum Beispiel 0,05 bis 5 mg, vorzugsweise 0,10 bis 3 mg, insbesondere 0,3 bis 2 mg Reproterol.

Für Fenoterol und Terbutalin kommen beispielsweise folgende Mengen in Frage: 0,02 bis 2, vorzugsweise 0,05 bis 1, insbesondere 0,1 bis 0,5 mg (Azelastinmenge wie zuvor angegeben)

Für Salbutamol kommen beispielsweise folgende Mengen in Frage: 0,01 bis 1, vorzugsweise 0,02 bis 0,5, insbesondere 0,05 bis 0,3 mg (Azelastinmenge wie zuvor angegeben).

Diese Dosen können beispielsweise 1 bis 10, vorzugsweise 1 bis 5, insbesondere 1 bis 3 mal täglich verabreicht werden.

Bei Verwendung mit Theophyllin kann die Dosierungseinheit der erfindungsgemäßen Kombination beispielsweise enthalten:

a) Bei peroralen Arzneiformen oder Arzneiformen zur rektalen oder vaginalen Applikation:

0,1 bis 30 mg Azelastin, vorzugsweise 0,5 bis 20 mg, insbesondere 1 bis 10 mg Azelastin und 100 bis 500 mg, vorzugsweise 100 bis 400 mg, insbesondere 100 bis 300 mg Theophyllin.

Diese Dosen können beispielsweise 1 bis 5, vorzugsweise 1 bis 4, insbesondere 1 bis 3 mal täglich verabreicht werden.

b) Bei parenteralen Arzneiformen (zum Beispiel intravenös, intramuskulär):

0,05 bis 30 mg Azelastin, vorzugsweise 0,1 bis 20 mg, insbesondere 0,3 bis 10 mg Azelastin und 10 bis 500 mg, vorzugsweise 30 bis 400 mg, insbesondere 50 bis 300 mg Theophyllin.

Diese Dosen können beispielsweise 1 bis 5, vorzugsweise 1 bis 4, insbesondere 1 bis 3 mal täglich verabreicht werden.

c) Bei Arzneiformen zur Applikation auf die Haut und Schleimhäute (zum Beispiel als Lösungen, Lotionen, Emulsionen, Salben, Pflaster und so weiter)

5 bis 50 mg Azelastin, vorzugsweise 5 bis 30 mg, insbesondere 10 bis 20 mg Azelastin und 100 bis 1000 mg, vorzugsweise 250 bis 750 mg, insbesondere 250 bis 700 mg Theophyllin.

Diese Dosen können beispielsweise 1 bis 5, vorzugsweise 1 bis 4, insbesondere 1 bis 3 mal täglich verabreicht werden.

Selbstverständlich können auch galenische Zubereitungen hergestellt werden, welche die angegebenen Dosierungseinheiten 2 bis beispielsweise 200 mal enthalten. Insbesondere enthalten Tabletten oder Kapseln der Kombination 0,2 bis 500 mg, Pellets, Pulver und Granulate 0,5 bis 100 mg, Dosier-Aerosole 0,5 bis 1000 mg der Azelastin-Komponente.

Die in den vorangegangenen Seiten angegebenen Dosen und Gewichtsteile, die sich auf die Anwendung am Menschen beziehen, sind jeweils bezogen auf die freien Basen, beziehungsweise freien Säuren.

Die akute Toxizität der erfindungsgemäßen Kombination an der Maus (ausgedrückt durch LD50 mg/kg; Methode: Litchfield und Wilcoxon, J. Pharmacol. Exper. Ther. 95 : 99, 1949) liegt beispielsweise für die Kombination Azelastin und Theophyllin (4:100) bei oraler Applikation bei 350 mg/kg beziehungsweise oberhalb von 300 mg/kg Körpergewicht.

Die akute Toxizität der erfindungsgemäßen Kombination an der Maus (ausgedrückt durch LD50 mg/kg; Methode: Litchfield und Wilcoxon, J. Pharmacol. Exper. Ther. 95 : 99, 1949) liegt beispielsweise für die Kombination Azelastin und Reproterol (1:5) bei oraler Applikation bei 1700 mg/kg beziehungsweise oberhalb von 1000 mg/kg Körpergewicht.

Die pharmazeutischen Zusammensetzungen beziehungsweise Arzneimittel enthalten als Wirkstoff die erfindungsgemäße Kombination in einer Formulierung. Die Einzelwirkstoffe der Kombination können aber auch in jeweils getrennten Formulierungen vorliegen, wobei die bereits angegebenen Wirkstoffmengen jeweils für die betreffende Dosierungseinheit verwendet werden. Die Wirkstoffe beziehungsweise die Wirkstoffkombination liegt gegebenenfalls in Mischung mit anderen pharmakologisch beziehungsweise pharmazeutisch wirksamen Stoffen vor. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rizinusöl, Olivenöl, Sesamöl, Baumwollsaatöl, Maisöl, Weizenkeimöl, Sonnenblumensamenöl, Kabeljau-Leberöl, jeweils auch hydriert; Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen und Essigsäure, Benzylbenzoat, Dioxolane, Glyzerinformale, Tetrahydrofurfurylalkohol, Polyglykolether mit $C_1$-$C_{12}$ Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie:
quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage:
Polymerisate sowie Copolymerisate oder Acrylsäure und/oder Methacrylsäure und/oder deren Ester; Hydroxypropylmethylcellulosephthalat oder -acetatsuccinat; Cellulose-, Stärke- sowie Polyvinylacetatphthalat; Carboxymethylcellulose; Methylcellulose-phthalat, -succinat, -phthalatsuccinat sowie - phthalsäurehalbester; Zein; Ethylcellulose sowie -succinat; Schellack; Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethyl-hexyl-acrylatmaleinsäureanhydrid; Crotonsäure-Vinylacetat-Copolymer; Glutaminsäure/Glutaminsäureester-Copolymer; Carboxymethylethylcelluloseglycerinmonooctanoat; Celluloseacetatsuccinat; Polyarginin.
Als Plastifizierungsmittel für Hüllstoffe können in Frage kommen:
Citronen- und Weinsäureester (Acetyltriethyl-, Acetyltributyl-, Tributyl-, Triethyl-citrat);
Glycerin und Glycerinester (Glycerindiacetat, -triacetat, acetylierte Monoglyceride, Rizinusöl);
Phthalsäureester (Dibutyl-, Diamyl-, Diethyl-, Dimethyl-, Dipropyl-), D-(2-Methoxy- oder -ethoxyethyl)-phthalat, Ethylphthalyl-, Butylphthalylethyl- und Butylglycolat; Alkohole (Propylenglykol, Polyethylenglykol verschiedener Kettenlängen), Adipate (Diethyl-, Di(2-Methoxy- oder ethoxyethyladipat); Benzophenon; Diethyl- und Dibutylsebacat, -succinat, -tartrat;
Diethylenglykoldipropionat; Ethylenglykoldiacetat, -dibutyrat, -dipropionat; Tributylphosphat, Tributyrin; Polyethylenglykolsorbitanmonooleat; Sorbitanmonooleat.

Zur Herstellung von Lösungen kommen beispielsweise Wasser oder physiologisch verträgliche organische Lösungsmittel in Frage, wie zum Beispiel Ethanol, 1,2-Propylenglykol, Polyglykole und deren Derivate, Dimethylsulfoxid, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Tragant, polyoxyethyliertes sorbitanmonooleat und andere ethoxylierte Fettsäureester des Sorbinat polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxyd pro Mol Glycerid).
Beispiele für Oleotriglyceride sind Olivenöl, Erdnussöl, Rizinusöl, Sesamöl, Baumwollsaatöl, Maisöl.
Siehe auch Dr. H P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Treibmittel für inhalativ applizierbare Dosier-Aerosole kommen beispielsweise in Frage: Fluorierte chlorierte Kohlenwasserstoffe wie Tr1chlorfluormethan, Dichlordifluormethan, Trichlortrifluorethan, symmetrisches Dichlortetrafluorethan, Dimethylether, Propan, Butan, Kohlendioxid.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.
Als Konservierungsmittel kommen beispielsweise Sorbinsäure, p-Hydroxybenzoesäureester (zum Beispiel Niederalkylester), Benzoesäure, Natriumbenzöat, Trichlorisobutylalkohol, Phenol, Kresol, Benzethoniumchlorid und Formalinderivate in Betracht.

Die pharmazeutische und galenische Handhabung der Wirkstoffe erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80° C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.
Bei den parenteralen Zubereitungsformen handelt es sich insbesondere um sterile beziehungsweise sterilisierte Erzeugnisse.

Die erfindungsgemäße Kombination kann auch als ein Erzeugnis vorliegen, bei dem jeweils die beiden Einzelwirkstoffe in getrennten Formulierungen vorliegen, so daß auch eine getrennte oder auch zeitlich abgestufte Verabreichung möglich ist.
Falls solche getrennten Formulierungen vorliegen, sind diese aufeinander abgestimmt und enthalten die jeweiligen Wirkstoffe in der Dosierungseinheit in den Mengen und entsprechenden Gewichtsverhältnissen, in denen sie in der kombinierten Mischung vorliegen können.

Bei getrennter Anwendung ist es auch möglich, daß beide Kombinationspartner nicht gleichzeitig verabreicht werden. In solchen Fällen kann das $\beta$-Mimetikum (zum Beispiel Reproterol) 5 bis 60 Minuten vor oder 5 bis 240 Minuten nach Verabreichung des Azelastins gegeben werden. Für die Kombination Azelastin Theophyllin gilt für diesen Fall folgendes:
Vorweggabe von Theophyllin 5 bis 60 Minuten vor Azelastin beziehungsweise 5 bis 240 Minuten nach Azelastin.

Beispiel 1

Tabletten mit 3 mg Azelastinhydrochlorid und 300 mg Theophyllin-Monohydrat beziehungsweise mit 1 mg Azelastinhydrochlorid und 100 mg Theophyllin-Monohydrat:

Aus 18 g Maisstärke und 333 g Wasser wird ein Schleim hergestellt, mit dem eine Mischung aus 9 g fein gepulvertem Azelastinhydrochlorid und 300 g mikrokristalliner Cellulose in der üblichen Weise granuliert wird. Nach dem Trocknen wird das Granulat durch ein Sieb der Maschenweite 1 mm gegeben und anschlieβend mit 900 g Theophyllin-Monohydrat, 180 g mikrokristalliner Cellulose, 86,1 g modifizierter Stärke (Starch 1500/Colorcon), 0,9 g Hochdispersem Siliciumdioxid und 6 g Magnesiumstearat gemischt. Die fertige Mischung wird zu Oblong-Tabletten vom Gewicht 500 mg, einer Länge von 18 mm und einer Breite Von 8 mm verpreßt.

Anschließend können die Tabletten gegebenenfalls in der üblichen Weise mit einem Filmüberzug versehen werden.

Eine Tablette enthält 3 mg Azelastinhydrochlorid und 300 mg Theophyllin-Monohydrat.

In ähnlicher Weise können Tabletten mit 1 mg Azelastinhydrochlorid und 100 mg Theophyllin-Monohydrat hergestellt werden, wenn aus der oben erwähnten fertigen Mischung Tabletten vom Gewicht 166,6 mg und einem Durchmesser von 8 mm gepreßt werden.

Beispiel 2

Tabletten mit 3 mg Azelastinhydrochlorid und 20 mg Reproterolhydrochlorid beziehungsweise mit 1,5 mg Azelastinhydrochlorid und 10 mg Reproterolhydrochlorid:

150 g Azelastinhydrochlorid werden mit 1000 g Reproterolhydrochlorid, 4000 g Milchzücker, 1000 g Maisstärke, 1000 g mikrokristalliner Cellulose und 90 g Hochdispersem Siliciumdioxid intensiv gemischt und anschließend mit 2,5 kg Maisstärkeschleim 8 % nach dem üblichen Verfahren granuliert. Das Granulat wird nach der Trocknung durch ein Sieb der Maschenweite 1 mm gegeben und mit 1500 g mikrokristalliner Cellulose, 525 g Maisstärke, 500 g Talkum, 10 g Hochdispersem Siliciumdioxid und 25 g Magnesiumstearat gemischt. Die fertige Mischung wird zu Oblong-Tabletten vom Gewicht 200 mg, einer Länge von 11 mm und einer Breite von 5,5 mm verpreßt.

Gegebenenfalls können die Tabletten nach dem üblichen Verfahren mit einem Filmüberzug versehen werden.

Eine Tablette enthält 3 mg Azelastinhydrochlorid und 20 mg Reproterolhydrochlorid.

In ähnlicher Weise können Tabletten mit 1,5 mg Azelastinhydrochlorid und 10 mg Reproterolhydrochlorid hergestellt werden, wenn aus der oben erwähnten fertigen Mischung Tabletten vom Gewicht 100 mg, einem Durchmesser von 6 mm und einem Wölbungsradius Von 4 mm verpreßt werden.

Beispiel 3

Suppositorien mit 3 mg Azelastinhydrochlorid und 100 mg Theophyllin-Monohydrat beziehungsweise 1 mg Azelastinhydrochlorid und 300 mg Theophyllin-Monohydrat:

15 g Azelastinhydrochlorid und 500 g Theophyllin-Monohydrat werden in 9,835 kg geschmolzenem Hartfett * (siehe Europäisches Arzneibuch, Band III) suspendiert. Nach Homogenisierung wird die Suspension in üblicher Weise in Hohlzellen von 2,3 ml ausgegossen und abgekühlt.

Ein Suppositorium vom Gewicht 2,07 g enthält 3 mg Azelastinhydrochlorid und 100 mg Theophyllin-Monohydrat.

In analoger Weise können Suppositorien mit 1 mg Azelastinhydrochlorid und 300 mg Theophyllin-Monohydrat hergestellt werden, wenn 5 g Azelastinhydrochlorid und 1500 g Theophyllin-Monohydrat in 9245 g geschmolzenem Hartfett suspendiert werden, sonst aber gleich gearbeitet wird.

Beispiel 4

Suppositorien mit 3 mg Azelastinhydrochlorid und 10 mg Reproterolhydrochlorid beziehungsweise 1 mg Azelastinhydrochlorid und 20 mg Reproterolhydrochlorid:

15 g Azelastinhydrochlorid und 50 g Reproterolhydrochlorid werden in 10,135 kg geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2,04 g enthält 3 mg Azelastinhydrochlorid und 10 mg Reproterolhydrochlorid.

In anlaloger Weise können Suppositorien mit 1 mg Azelastinhydrochlorid und 20 mg Reproterolhydrochlorid hergestellt werden, wenn 5 g Azelastinhydrochlorid und 100 g Reproterolhydrochlorid in 10,095 kg geschmolzenem Hartfett suspendiert werden, ansonsten aber gleich gearbeitet wird.

* Hartfett ist ein Gemisch von Mono-, Di- und Triglyceriden der gesättigten Fettsäuren von $C_{10}H_{20}O_2$ bis $C_{18}H_{36}O_2$

Beispiel 5
Dosieraerosol mit 1,0 mg Azelastinhydrochlorid und 0,5 mg Reproterolhydrochlorid pro Hub:

450 g Dichlordifluormethan werden auf etwa -50° C abgekühlt und darin 14 g Sorbitantrioleat suspendiert. In die Suspension werden unter intensivem Rühren 20 g Azelastinhydrochlorid und 10 g Reproterolhydrochlorid portionsweise eingegeben. Die erhaltene Suspension wird mit Dichlordifluormethan auf 859,6 g aufgefüllt. Sodann werden 526,4 g 1,2-Dichlortetrafluorethan unter Rühren und Kühlen zugemischt. Unter weiterem Rühren wird die Suspension auf ca. -50° C abgekühlt und unter ständigem Rühren zu 13,86 g in Aluminiumdosen abgefüllt, die anschließend mit handelsüblichen Dosierventilen verschlossen werden (zum Beispiel mit Dosierventilen aus Metall der Firma Riker /3 M). Die Dosierventile setzen pro Hub 0,05 ml Aerosolsuspension frei. Pro Hub werden damit 1 mg Azelastinhydrochlorid und 0,5 mg Reproterolhydrochlorid freigesetzt.

Beispiel 6
Retardtabletten mit 400 mg Theophyllin-Monohydrat und 5 mg Azelastinhydrochlorid

5 g Azelastinhydrochlorid werden stufenweise mit 200 g Hydroxypropyl-cellulose (Viskosität einer 2%igen wässrigen Lösung: 150 - 400 cps) und 200 g Hydroxypropylcellulose (Viskosität einer 2%igen wässrigen Lösung: 6 - 10 cps) sowie 400 g Theophyllin-Monohydrat intensiv gemischt und die Mischung in bekannter Weise trocken verpreßt. Nach Aufmahlung des so erhaltenen Produktes (Komprimat) auf eine Korngröße unter 0,4 mm werden 5 g Magnesiumstearat zugemischt und die erhaltene Mischung zu Oblong-Tabletten vom Gewicht 810 mg, einer Länge von 19 mm und einer Breite von 8 mm gepreßt. Anschließend könne die Tablette gegebenenfalls in der üblichen Weise mit einem magensaftlöslichen Filmüberzug versehen werden.

Eine Tablette enthält 400 mg Theophyllin-Monohydrat und 5 mg Azelastinhydrochlorid in einer Zubereitung mit verzögerter Wirkstoffgabe.

Beispiel 7
Hartgelatinekapseln mit 350 mg Theophyllin-Monohydrat in Retardzubereitung und 5 mg Azelastinhydrochlorid

400 g Theophyllin-Monohydrat werden mit 10 g mikrokristalliner Cellulose und 7 g Hydroxypropylcellulose (Viskosität der 5%igen Lösung 75 - 150 cps) gemischt und die Mischung mit 60 g einer 6,25%igen wässrigen Lösung von Hydroxypropylcellulose angeteigt. Die feuchte Masse wird durch ein übliches Lochblech mit einem Lochdurchmesser von 1mm gepreßt und die entstehenden Stränge durch Behandlung auf einer Spheronizer-Scheibe in üblicher Weise zerteilt und ausgerundet. Die erhaltenen Pellets werden getrocknet und gesiebt. 400 g Pellets der Siebfraktion 800 - 1200 µm werden mit einer Lösung aus 42,5 g Ethylcellulose und 37,5 g Polyethylenglykol 1500 in 720 g Chloroform durch Besprühen in der Wirbelschicht-Apparatur in üblicher Weise überzogen.

70 g Azelastinhydrochlorid werden mit 400 g Milchzucker, 97 g Maisstärke, 100 g mikrokristalliner Cellulose und 9 g hochdispersem Siliciumdioxid intensiv gemischt und anschließend mit 250 g Maisstärkeschleim 8 % nach dem üblichen Verfahren granuliert. Nach der Trocknung wird das Granulat durch einen Sieb der Maschenweite 1 mm gegeben und mit 3 g Magnesiumstearat sowie 1 g hochdispersem Siliciumdioxid gemischt.

Die erhaltene Mischung wird in einer Menge von 50 mg zusammen mit jeweils 465 mg der oben erhaltenen überzogenen Pellets in Hartgelatinekapseln der Größe 0 gefüllt.

Eine Hartgelatinekapsel enthält 350 mg Theophyllin-Monohydrat in Retardzubereitung und 5 mg Azelastinhydrochlorid.

Beispiel 8
Retardtabletten mit 30 mg Reproterolhydrochlorid und 5 mg Azelastinhydrochlorid

600 g Reproterolhydrochlorid, 100 g Azelastinhydrochlorid, 2200 g Hydroxypropylmethylcellulose (Viskosität einer 2%igen wässrigen Lösung: 4000 cP), 2600 g sprühgetrocknete Lactose und 60 g Magnesiumstearat werden gemischt und die Mischung zu runden Tabletten vom Gewicht 278 mg , einem Durchmesser von 9 mm und einem Wölbungsradius von 9 mm verpreßt.
Im Anschluß daran können die Tabletten nach üblichen Verfahren mit einem magensaftlöslichen Filmüberzug versehen werden.

Eine Retardtablette enthält 30 mg Reproterolhydrochlorid und 5 mg Azelastinhydrochlorid.

Beispiel 9
Zubereitungen unter Verwendung weiterer ß-Mimetika

a) Retardtabletten mit 7,5 mg Terbutalinsulfat und 5 mg Azelastinhydrochlorid

150 g Terbutalinsulfat, 100 g Azelastinhydrochlorid, 960 g Hydroxypropylmethylcellulose * , 1170 g sprühgetrocknete Lactose und 20 g Magnesiumstearat werden gemischt und die Mischung zu runden Tabletten vom Gewicht 120 mg, einem Durchmesser von 6 mm und einem Wölbungsradius von 6 mm verpreßt.

Im Anschluß daran können die Tabletten nach üblichen Verfahren mit einem magensaftlöslichen Filmüberzug versehen werden.

Eine Retardtablette enthält 7,5 mg Terbutalinsulfat und 5 mg Azelastinhydrochlorid.
b) Retardtabletten mit 10 mg Salbutamolsulfat und 5 mg Azelastinhydrochlorid

Es wird wie unter a) angegeben ist gearbeitet, jedoch werden statt 150 g Terbutalinsulfat 200 g Salbutamolsulfat und statt 1170 g sprühgetrockneter Lactose 1120 g sprühgetrocknete Lactose eingesezt.
c) Retardtabletten mit 5 mg Fenoterolhydrobromid und 5 mg Azelastinhydrochlorid

Es wird wie unter a) angegeben ist gearbeitet, jedoch werden statt 150 g Terbutalinsulfat 100 g Fenoterolhydrobromid und statt 1170 g sprühgetrockneter Lactose 1220 g sprühgetrocknete Lactose eingesetzt.

**Patentansprüche**

**1.** Erzeugnisse, enthaltend als Wirkstoff Azelastin und Theophyllin oder Azelastin und mindestens ein ß-Mimetikum oder Salze dieser Verbindungen mit physiologisch unbedenklichen Säuren als synergistisch wirkendes Kombinationspräparat zur gleichzeitigen oder getrennten Anwendung, wobei auf 1 Gewichtsteil Azelastin 0,0003 bis 1000 Gewichtsteile ß-Mimetikum oder 3 bis 5000 Gewichtsteile Theophyllin verwendet werden.

**2.** Erzeugnisse nach Anspruch 1,
dadurch gekennzeichnet,
daß in der Dosierungseinheit für die Kombination 0,1 bis 50 mg, vorzugsweise 0,2 bis 20 mg Azelastin und entweder 10 bis 1000 mg, vorzugsweise 80 bis 600 mg Theophyllin oder 0,005 bis 200 mg ß-Mimetikum verwendet werden.

**3.** Verfahren zur Herstellung eines injizierbaren Erzeugnisses nach einem oder mehreren der vorangegangenen Ansprüche,
dadurch gekennzeichnet,
daß man 1 Gewichtsteil Azelastin und 0,0003 bis 1000 Gewichtsteile ß-Mimetikum oder 1 Gewichtsteil Azelastin und 3 bis 5 000 Gewichtsteile Theophyllin, wobei die Wirkstoffe auch in Form von Salzen mit physiologisch unbedenklichen Säuren vorliegen können, zusammen mit üblichen Träger- und/oder Verdünnungs- beziehungsweise Hilfsstoffen zu einem Mittel verarbeitet, welches in der Dosierungseinheit 0,1 bis 50 mg Azelastin und 0,01 bis 200 mg ß-Mimetikum oder 0,05 bis 50 mg Azelastin und 10 bis 500 mg Theophyllin enthält.

**4.** Verfahren zur Herstellung eines peroral oder rektal zu verabreichenden Erzeugnisses nach einem oder mehreren der vorangegangenen Ansprüche
dadurch gekennzeichnet,
daß man 1 Gewichtsteil Azelastin und 0,0003 bis 1000 Gewichtsteile ß-Mimetikum oder 1 Gewichtsteil Azelastin und 3 bis 5000 Gewichtsteile Theophyllin, wobei die Wirkstoffe auch in Form von Salzen mit

*) Viskosität einer 2%igen wässrigen Lösung: 4000 cP

physiologisch unbedenklichen Säuren vorliegen können, zusammen mit üblichen Träger-und/oder Verdünnungs- beziehungsweise Hilfsstoffen bei Temperaturen zwischen 0 und 80°C vermischt beziehungsweise homogenisiert, die so erhaltene Mischung zur Herstellung von Zubereitungen, die in der Dosierungseinheit 0,1 bis 30 mg Azelastin und 0,01 bis 200 mg ß-Mimetikum oder 0,1 bis 30 mg Azelastin und 100 bis 500 mg Theophyllin enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt, oder in Kapseln abfüllt.

5. Verfahren zur Herstellung eines Erzeugnisses nach einem oder mehreren der vorangegangenen Ansprüche,
dadurch gekennzeichnet,
daß man 1 Gewichtsteil Azelastin und 0,0003 bis 1000 Gewichtsteile ß-Mimetikum oder 1 Gewichtsteil Azelastin und 3 bis 5000 Gewichtsteile Theophyllin, wobei die Wirkstoffe auch in Form von Salzen mit physiologisch unbedenklichen Säuren vorliegen können, gegebenenfalls mit mindestens einem der Hilfsstoffe Stärke, Cellulose, Calciumhydrogenphosphat und modifizierte Stärke vermischt, mit einer wäßrigen Gelatinelösung oder Stärkelösung oder einem wäßrigen Vinylpyrrolidon-Vinylacetat Copolymerisat granuliert und das erhaltene Granulat mit Magnesiumstearat und hochdispersem Siliziumdioxyd sowie gegebenenfalls auch Stärke und/oder Cellulose vermischt und zu Tabletten verpreßt oder in Kapseln abfüllt.

6. Verfahren zur Herstellung eines Mittels nach einem oder mehreren der vorangegangenen Ansprüche,
dadurch gekennzeichnet,
daß man 1 Gewichtsteil Azelastin und 0,0003 bis 1000 Gewichtsteile ß-Mimetikum oder 1 Gewichtsteil Azelastin und 3 bis 5000 Gewichtsteile Theophyllin, wobei die Wirkstoffe auch in Form von Salzen mit physiologisch unbedenklichen Säuren vorliegen können, gegebenenfalls nach Zusatz von Sojalecithin, bei Temperaturen zwischen 33 bis 37°C in geschmolzenem Hartfett supendiert und homogenisiert und anschließend die Mischung in Hohlzellen ausgießt.

7. Verwendung von Azelastin und Reproterol beziehungsweise Azelastin und Theophyllin zur Herstellung von synergistisch wirkenden Mitteln zur Bekämpfung des Asthma bronchiale und der chronisch obstruktiven Bronchitis.

**Claims**

1. Products containing as active principle azelastine and theophylline or azelastine and at least one $\beta$-mimetic or salts of these compounds with physiologically safe acids as a synergistic compound preparation for simultaneous or separate application, 0.0003 to 1,000 parts by weight of the $\beta$-mimetic or 3 to 5,000 parts by weight theophylline being used to 1 part by weight azelastine.

2. Products as claimed in claim 1, characterized in that 0.1 to 50 mg and preferably 0.2 to 20 mg azelastine and either 10 to 1,000 mg and preferably 80 to 600 mg theophylline or 0.005 to 200 mg of the $\beta$-mimetic are used in the dosage unit for the combination.

3. A process for the preparation of an injectable product as claimed in one or more of the preceding claims, characterized in that 1 part by weight azelastine and 0.0003 to 1,000 parts by weight of the $\beta$-mimetic or 1 part by weight azelastine and 3 to 5,000 parts by weight theophylline - the active substances may also be present in the form of salts with physiologically safe acids - are processed together with typical excipients and/or diluents or auxiliaries to form a preparation containing 0.1 to 50 mg azelastine and 0.01 to 200 mg $\beta$-mimetic or 0.05 to 50 mg azelastine and 10 to 500 mg theophylline.

4. A process for the production of a perorally or rectally applied product as claimed in one or more of the preceding claims, characterized in that 1 part by weight azelastine and 0.0003 to 1,000 parts by weight of the $\beta$-mimetic or 1 part by weight azelastine and 3 to 5,000 parts by weight theophylline - the active substances may also be present in the form of salts with physiologically safe acids - are mixed or homogenized with typical excipients and/or diluents or auxiliaries at temperatures of 0 to 80°C, the resulting mixture - to form preparations containing 0.1 to 30 mg azelastine and 0.01 to 200 mg $\beta$-mimetic or 0.1 to 30 mg azelastine and 100 to 500 mg theophylline - is poured into hollow cells of

appropriate size or is introduced into capsules of appropriate size or is granulated and then pressed to tablets, optionally with addition of other typical auxiliaries, or introduced into capsules.

5. A process for the preparation of a product as claimed in one or more of the preceding claims, characterized in that 1 part by weight azelastine and 0.0003 to 1,000 parts by weight of the β-mimetic or 1 part by weight azelastine and 3 to 5,000 parts by weight theophylline - the active substances may also be present in the form of salts with physiologically safe acids - are mixed, optionally with at least one of the auxiliaries starch, cellulose, calcium hydrogen phosphate and modified starch, the resulting mixture is granulated with an aqueous gelatine solution or starch solution or an aqueous vinyl pyrrolidone/vinyl acetate copolymer and the granules obtained are mixed with magnesium stearate and highly disperse silicon dioxide and, optionally, starch and/or cellulose and the resulting mixture is pressed to tablets or introduced into capsules.

6. A process for the preparation of a product as claimed in one or more of the preceding claims, characterized in that 1 part by weight azelastine and 0.0003 to 1,000 parts by weight of the β-mimetic or 1 part by weight azelastine and 3 to 5,000 parts by weight theophylline - the active substances may also be present in the form of salts with physiologically safe acids - are suspended and homogenized in molten hard fat at temperatures of 33 to 37° C, optionally after addition of soya lecithin, and the mixture is subsequently poured out into hollow cells.

7. The use of azelastine and reproterol or azelastine and theophylline for the production of synergistic preparations for treating bronchial asthma and chronic obstructive bronchitis.

**Revendications**

1. Produits contenant, en tant que substance active, de l'azélastine et de la théophylline ou de l'azélastine et au moins un β-mimétique ou des sels de ces composés avec des acides physiologiquement inoffensifs, utilisables comme préparation combinée à action synergique pour l'administration simultanée ou séparée, de 0,0003 à 1000 parties en poids de β-mimétique ou de 3 à 5000 parties en poids de théophylline étant utilisées pour 1 partie en poids d'azélastine.

2. Produits selon la revendication 1, caractérisés en ce qu'il est utilise, pour la combinaison dans l'unité posologique, de 0,1 à 50 mg, de préférence de 0,2 à 20 mg d'azélastine et de 10 à 1000 mg, de préférence de 80 à 600 mg de théophylline ou de 0,005 à 200 mg de β-mimétique.

3. Procédé de préparation d'un produit injectable selon la revendication 1 ou 2, caractérisé en ce qu'on travaille avec des excipients et/ou des diluants ou adjuvants usuels 1 partie en poids d'azélastine et de 0,0003 à 1000 parties en poids d'un β-mimétique ou 1 partie en poids d'azélastine et de 3 à 5000 parties en poids de théophylline, les substances actives pouvant aussi se présenter sous forme de sels avec des acides physiologiquement inoffensifs, pour obtenir un médicament qui contient, dans l'unité posologique, de 0,1 à 50 mg d'azélastine et de 0,01 à 200 mg de β-mimétique ou de 0,05 à 50 mg d'azélastine et de 10 à 500 mg de théophylline.

4. Procédé de préparation d'un produit à administrer par voie orale ou rectale selon l'une quelconque des revendications 1 à 3, caractérisé en ce qu'on mélange ou homogénéise à une température comprise entre 0 et 80° C, en combinaison avec des excipients et/ou diluants ou adjuvants usuels, 1 partie en poids d'azélastine et de 0,0003 à 1000 parties en poids d'un β-mimétique ou 1 partie en poids d'azélastine et de 3 à 5000 parties en poids de théophylline, les substances actives pouvant aussi se présenter sous forme de sels avec des acides physiologiquement inoffensifs, on verse le mélange ainsi obtenu dans des cellules creuses de dimensions appropriées, on en remplit des capsules de taille appropriée ou on le granule puis on le presse en comprimés, éventuellement avec addition d'autres adjuvants usuels, ou on en remplit des capsules, pour obtenir des préparations qui contiennent, dans l'unité posologique, de 0,1 à 30 mg d'azélastine et de 0,01 à 200 mg de β-mimétique ou de 0,1 à 30 mg d'azélastine et de 100 à 500 mg de théophylline.

5. Procédé de préparation d'un produit selon l'une quelconque des revendications 1 à 3, caractérisé en ce qu'on mélange 1 partie en poids d'azélastine et de 0,0003 à 1000 parties en poids d'un β-mimétique ou 1 partie en poids d'azélastine et de 3 à 5000 parties en poids de théophylline, les substances

actives pouvant aussi se présenter sous forme de sels avec des acides physiologiquement inoffensifs, éventuellement avec l'un au moins des adjuvants amidon, cellulose, hydrogénophosphate de calcium et amidon modifié, on granule le mélange avec une solution aqueuse de gélatine ou d'amidon ou avec un copolymère vinylpyrrolidone/acétate de vinyle aqueux, on mélange le granulé obtenu avec du stéarate de magnésium et du dioxyde de silicium fortement dispersé, ainsi éventuellement qu'avec de l'amidon et/ou de la cellulose, et on le presse en comprimés ou on en remplit des capsules.

**6.** Procédé de préparation d'un médicament selon l'une quelconque des revendications 1 à 5, caractérisé en ce qu'on met en suspension et on homogénéise dans de la graisse dure fondue, à une temperature comprise entre 33 et 37 °C, éventuellement après addition de lécithine de soja, 1 partie en poids d'azelastine et de 0,0003 à 1000 parties en poids d'un β-mimétique ou 1 partie en poids d'azélastine et de 3 à 5000 parties en poids de théophylline, les substances actives pouvant aussi se présenter sous forme de sels avec des acides physiologiquement inoffensifs, puis on verse le mélange dans des cellules creuses.

**7.** Utilisation d'azélastine et de reprotérol ou d'azélastine et de théophylline pour la préparation de médicaments à action synergique pour lutter contre l'asthme bronchique et la bronchite chronique obstructive.